# NEUE EUROPÄISCHE PATENTSCHRIFT

(11) **EP 0 395 857 B2**
(45) Veröffentlichungstag und Bekanntmachung des Hinweises auf die Entscheidung über den Einspruch: **16.12.1998**
(45) Hinweis auf die Patenterteilung: 17.08.1994
(21) Anmeldenummer: 90104478.4
(22) Anmeldetag: 09.03.1990
(51) Int. Cl.: C07C 2/12, C07C 7/13

(54) **Verfahren zur Oligomerisierung von Olefinen**
Process for oligomerising olefins
Procédé pour oligomérisation d'oléfines

(30) Priorität: 05.05.1989 DE 3914817
(43) Veröffentlichungstag der Anmeldung: 07.11.1990
(73) Patentinhaber: HÜLS AKTIENGESELLSCHAFT, 45764 Marl (DE)
(72) Erfinder: Nierlich, Franz, Dr., D-4370 Marl (DE); Neumeister, Joachim, Dr., D-4370 Marl (DE); Wildt, Thomas, Dr., D-4358 Haltern (DE); Droste, Wilhelm, Dr., D-4370 Marl (DE); Obenaus, Fritz, Dr., D-4370 Marl (DE)
(74) Vertreter: Patentanwälte Sternagel & Fleischer

(56) Entgegenhaltungen:
- EP-A- 0 229 994
- EP-A- 0 281 208
- DD-A- 152 331
- DD-C- 160 037
- DE-A- 2 057 269
- DE-A- 2 143 759
- DE-A- 3 611 853
- FR-A- 2 556 715
- GB-A- 2 154 603
- US-A- 2 828 347
- US-A- 2 906 790
- US-A- 3 816 975
- US-A- 4 153 638
- Chemie in unserer Zeit 20 (1986) Seite 117.
- Ullmanns Encyklopädie der tech. Chemie, 4. Aufl., Bd. 13, S. 544-545, Verlag Chemie, Weinheim, DE, 1977

## Beschreibung

Die Erfindung betrifft ein Verfahren zur Oligomerisierung von Olefinen mit 2 bis 8 Kohlenstoffatomen oder deren Mischungen, die in einem Kohlenwasserstoff-Gemisch enthalten sind, an einem nickelhaltigen Katalysator bei Temperaturen von 0 bis 200 °C und bei Drücken von 1 bis 70 bar abs.

Olefine mit 2 bis 8 Kohlenstoffatomen oder deren Gemische, insbesondere Olefine mit zwei bis fünf Kohlenstoffatomen, stehen in großen Mengen aus Raffineriecrackern zur Verfügung und stellen deshalb wichtige Rohstoffe für die gesamte Petrochemie dar. So haben die Polymeren von Äthylen, Propylen und Buten-1 oder Mischungen derselben zahlreiche Anwendungen gefunden.

Aber auch die durch Säurekatalyse hergestellten, verzweigten Oligomeren von Olefinen mit 2 bis 8 Kohlenstoffatomen haben großtechnische Bedeutung erlangt. So wird bereits seit Jahrzehnten die Herstellung von Polymerbenzin aus C₃/C₄-Olefingemischen praktiziert und verschiedene, daraus isolierte Fraktionen werden als Ausgangsmaterialien für z. B. PVC-Weichmacher oder Detergentien verwendet.

Bedeutender als die aus Polymerbenzinfraktionen hergestellten petrochemischen Produkte sind die aus lineareren Olefinoligomeren erzeugten, weil z. B. solche Waschrohstoffe oder Detergentien biologisch besser abbaubar sind oder solche PVC-Weichmacher u. a. niedrigere Viskositäten und verbesserte Tieftemperatureigenschaften bei jedoch vergleichbarem Dampfdruck aufweisen. Weniger verzweigte Oligomere mit 10 bis 16 Kohlenstoffatomen aus C₅/C₆-Olefinschnitten eignen sich hervorragend als Dieselkraftstoffkomponente. Dieser Aspekt ist in Ländern, die ihre Kraftstoffversorgung hauptsächlich auf Kohle ausrichten, von großer Wichtigkeit.

Die lineareren Oligomeren aus niedrigeren Olefinen sind zugänglich durch Umsetzung derselben bei Temperaturen von etwa 0 bis 200 °C und Drücken von ca. 1 bis 70 bar an sowohl homogenen als auch heterogenen Katalysatoren, die als aktive Komponente überwiegend Nickel enthalten. Es sind aber auch andere katalytisch aktive Metalle, wie z. B. Ruthenium (G. Braca, G. Slzana; La Chimica e l'Industria, 56 (1974), 110-116), Palladium gemäß US-PS 4 436 946 und Kupfer, Kobalt, Eisen, Chrom und Titan gemäß GB-PS 824 002 bekannt. Technische Bedeutung haben allerdings nur die nickelhaltigen Katalysatoren erlangt.

DE-PS 28 55 423 offenbart als homogenen Katalysator ein System, das aus dem Nickel-II-salz der Oktansäure, Ethylaluminiumdichlorid und einer freien Fettsäure besteht. Ein Katalysatorsystem dieser Art wird auch beim einzigen homogen katalysierten Verfahren von technischer Bedeutung zur Olefinoligomerisierung (DIMERSOL^{R}) (Y. Chauvin et. al., Chemistry and Industry, 1974, 375-378) eingesetzt.

Homogen katalysierte Verfahren zur Oligomerisierung von Olefinen sind wegen der technisch aufwendigen Abtrennung des Katalysatorsystems sehr kostenintensiv und erfordern darüber hinaus eine nicht einfache Entsorgung des zwangsweise anfallenden Abfallproduktes, das bei der Vernichtung des Katalysators entsteht.

Neben homogenen Katalysatoren sind auch zahlreiche heterogene Katalysatoren auf Basis Nickel und Silizium beschrieben, die oftmals zusätzlich noch Aluminium enthalten und auf unterschiedlichste Weise hergestellt werden. DD-PS 160 037 offenbart beispielsweise die Herstellung eines Ni Al-Fällungskatalysators auf SiO₂ als Trägermaterial. Andere Katalysatoren werden erhalten, indem man die an der Trägeroberfläche befindlichen positiv geladenen Teilchen, wie Protonen oder Natriumionen, gegen Nickelionen austauscht. Dabei kommen unterschiedlichste Trägermaterialien zum Einsatz. z. B. gemäß R. Espinoza et. al.; Appl. Cat. 31 -(1987), S. 259-266, amorphes Aluminiumsilikat, gemäß DE-PS 20 29 624 kristallines Aluminiumsilikat, gemäß NL-PS 8 500 459 Zeolithe vom ZSM-Typ, gemäß DE-PS 23 47 235 ein X-Zeolith, gemäß A. Barth et. al., Z. Anorg. Allg. Chem. 521 (1985), 207-214, X- und Y-Zeolithe und gemäß EP-A 0 233 302 ein Mordenit.

Es ist bekannt, daß nickelhaltige Katalysatoren empfindlich auf die verschiedensten Katalysatorgifte reagieren. Solche Katalysatorgifte sind u. a. mehrfach ungesättigte Kohlenwasserstoffe, wie z. B. Propin oder Butadien, Halogenverbindungen, Sauerstoffverbindungen, wie z. B. Wasser oder Alkohole, Schwefelverbindungen, wie z. B. Schwefelwasserstoff, Kohlenoxisulfid, Thioalkohole und Thioether sowie Stickstoffverbindungen, wie z. B. Amine, die im FCC-C₄-Kohlenwasserstoffschnitt (FCC ist die Abkürzung für Fluid Cat. Cracker) enthalten sind, oder Spuren an Butadienextraktionsmitteln, wie z. B. Acetonitril oder N-Methylpyrolidon im SC-Raffinat-I. (SC ist die Abkürzung für Steam Cracker).

Die Wirkungsweise dieser Katalysatorgifte ist hingegen nicht genau bekannt, man vermutet aber, daß sie auf eine stärkere Adsorption derselben als die der zu oligomerisierenden Olefine an den katalytisch aktiven Zentren zurückzuführen ist. Durch die Anwesenheit solcher Katalysatorgifte in den zu oligomerisierenden Olefinen nimmt die Katalysatoraktivität im Laufe der Zeit ab.

So sind aus dem Stand der Technik Verfahren bekannt, um verschiedene Verbindungen aus einem Kohlenwasserstoff-Gemisch zu entfernen: Polyene werden gemäß DE-PS 20 57 269 vor der Oligomerisierung bevorzugt bis zu 75 % zu den entsprechenden Monoolefinen katalytisch hydriert. US-PS 4 153 638 lehrt, daß der Diolefingehalt nach katalytischer Hydrierung unter 1 Gew.-% liegen soll.

EP-A 0 281 208 offenbart ein Zwei-Stufen-Verfahren zur Oligomerisierung von Olefinen, wobei in der ersten Stufe das olefinhaltige Einsatzkohlenwasserstoffgemisch unter im wesentlichen nicht-Oligomerisierungsbedingungen bei Temperaturen von 20 bis 150 °C über einen Katalysator mit wenigstens einem Metall, ausgewählt aus den Gruppen I a, I b, II a, II b, IV b, V b, VI b und VIII, geleitet und wobei in der zweiten Stufe bei einer Temperatur, die wenigstens um 50 °C über der der ersten Stufe liegt, das Einsatzkohlenwasserstoffgemisch anschließend an einem Katalysator vom Mordenit-Typ mit wenigstens einem Metall, ausgewählt aus den Gruppen I b, II a, II b, IV b, V b, VI b und VIII, oligomerisiert wird. Hierbei dient das nicht oligomerisierungsaktive Adsorptionsbett in der ersten Verfahrensstufe der Adsorption und/oder Umwandlung von im wesentlichen hochreaktiven Dienen.

Gemäß US-PS 2 906 790 können konjugierte Diolefine an einem Nickelkatalysator durch Hydrierung aus einem Olefine enthaltenden Kohlenwasserstoffgemisch entfernt werden, wobei allerdings hohe Verluste an Monoolefinen auftreten. Die so gereinigten Olefine sollen als Einsatzstoff für die Oxosynthese verwendet werden.

Da die nickelhaltigen Oligomerisierungskatalysatoren im allgemeinen auch hydrieraktiv sind, kann man gemäß EP-PS 0 091 232 die Diolefine durch Überleiten des mit Wasserstoff gesättigten Einsatz-Kohlenwasserstoff-Gemisches über den Oligomerisierungskatalysator in die entsprechenden Olefine überführen.

Im Einsatz-Kohlenwasserstoff-Gemisch gelöstes Wasser kann gemäß US-PS 4 153 638 durch übliche Trocknungsmittel, wie z. B. ein Molekularsieb mit einem Porendurchmesser von 3 Angström oder aktivierten Bauxit auf Restgehalte von weniger als 10 Gew.-ppm entfernt werden. Andere höhersiedende Sauerstoffverbindungen werden gemäß DE-PS 20 57 269 bei der Destillation des Kohlenwasserstoff-Gemisches als Sumpfprodukt abgezogen.

Schwefelverbindungen können durch eine Alkali- und Stickstoffverbindungen durch eine Wasserwäsche entfernt werden, wobei gemäß US-PS 4 153 638 bevorzugt Kohlenwasserstoff-Gemische mit Restgehalten von weniger als 1 Gew.-ppm Schwefel und 0,3 Gew.-ppm Stickstoff zur Oligomerisierung eingesetzt werden.

Die Verfahren gemäß dem Stand der Technik eignen sich aber nur zur groben Entfernung solcher Verbindungen.

Es enthält beispielsweise ein in Raffinerien an fallendes Propen/Propan-Gemisch (ca. 75 Gew.-% Propen und ca. 25 Gew.-% Propan) auch noch nach Entschwefelung gemäß dem Stand der Technik hochsiedende Schwefelverbindungen, wie z. B. Dimethylsulfid (Kp: 38 °C) oder Dimethyldisulfid (Kp: 109 °C), in Konzentrationen von kleiner als 0,5 Gew.-ppm. Diese Mengen an Schwefelverbindungen reichen aber bereits, um die Lebensdauer des Oligomerisierungskatalysators bis zur Unwirtschaftlichkeit hin zu vermindern.

Als weiteres Beispiel seien C₄-Kohlenwasserstoffschnitte genannt, die trotz ihrer vorherigen Destillation im Spurenbereich höhersiedende Sauerstoffverbindungen, wie z. B. Methyl-tert.-butylether, tert.-Butylalkohol oder Aceton, enthalten können. Ähnliches trifft auch auf C₅-Kohlenwasserstoff-Schnitte, wie z. B. Pyrolysebenzin, zu.

Da Katalysatorgifte aber schon in Spuren wirksam sind, reichen zu ihrer Entfernung aus dem Einsatz-Kohlenwasserstoff-Gemisch für die Oligomerisierung von Olefinen die obengenannten Grobreinigungs-Verfahren gemaß dem Stand der Technik nicht aus. Aus diesem Grunde haben sich in der Praxis die katalytischen Verfahren zur Oligomerisierung von Olefinen, insbesondere die heterogen katalysierten Verfahren, wegen zu geringer Katalysatorlebensdauer nicht durchsetzen können.

Der Erfindung liegt daher die Aufgabe zugrunde, ein wirtschaftlicheres Verfahren zur Oligomerisierung von Olefinen zu entwickeln, in welchem etwaige Katalysatorgifte so weitgehend aus dem Einsatz-Kohlenwasserstoff-Gemisch vor der Oligomerisierung entfernt werden, daß der nickelhaltige Katalysator eine gute Standzeit aufweist.

Überraschenderweise verlängert sich durch Überleiten des Einsatz-Kohlenwasserstoff-Gemisches über ein Molekularsieb mit einem Porendurchmesser von größer als 4 Angström bis 15 Angström und durch eine selektive Hydrierung der mehrfach ungesättigten Kohlenwasserstoffe die Lebensdauer des nickelhaltigen Katalysators so nachhaltig, daß jetzt auch der großtechnische Einsatz des katalytischen Verfahrens zur Oligomerisierung von Olefinen in wirtschaftlicher Weise möglich ist.

Gegenstand der vorliegenden Erfindung ist daher ein Verfahren zur Oligomerisierung von Olefinen mit 2 bis 8 Kohlenstoffatomen oder deren Mischungen, die in einem Kohlenwasserstoff-Gemisch enthalten sind, in der Flüssigphase an einem heterogenen nickelhaltigen Katalysator bei Temperaturen von 0 bis 200 °C und bei Drucken von 1 bis 70 bar abs.,
dadurch gekennzeichnet,
daß man das Einsatz-Kohlenwasserstoff-Gemisch vor der Oligomerisierung bei Drucken von 1 bis 200 bar abs. und bei Temperaturen von 0 bis 200 °C über ein Molekularsieb mit einem Porendurchmesser von größer als 4 Angstrom bis 15 Angström leitet und die mehrfach ungesättigten Kohlenwasserstoffe aus dem Einsatz-Kohlenwasserstoff-Gemisch vor der Oligomerisierung durch eine selektive Hydrierung entfernt, wobei aus dem Einsatz-Kohlenwasserstoff-Gemisch vor dem Überleiten desselben über das Molekularsieb mit einem Porendurchmesser von größer als 4 Angström bis 15 Angström im wesentlichen Wasser, Alkohole, Stickstoff-, Schwefel- und Halogenverbindungen entfernt werden.

Die Olefine im Einsatz-Kohlenwasserstoff-Gemisch werden an einem heterogenen nickelhaltigen Katalysator oligomerisiert. Bevorzugt werden sie an einem nickelhaltigen Katalysatorfestbett und besonders bevorzugt an einem nickel-, silicium- und aluminiumhaltigen Katalysatorfestbett oligomerisiert.

Des weiteren werden die Olefine im Einsatz-Kohlenwasserstoff-Gemisch in der Flüssigphase, oligomerisiert.

Es können als Molekularsiebe sowohl kristalline, natürliche Aluminiumsilikate, wie z. B. Schichtgittersilikate, als auch synthetische erfindungsgemäß eingesetzt werden.
Geeignet für das erfindungsgemäße Verfahren sind auch kommerzielle Molekularsiebe, wie z. B. Typen der Firmen Bayer AG, Dow, Union Carbide, Laporte oder Mobil. Diese Molekularsiebe können z. B. Zeolithe vom A-, X- und Y-Typ sein.

Des weiteren sind auch synthetische Molekularsiebe geeignet, die neben Silizium und Aluminium als Hauptbestandteile noch andere Atome als Nebenbestandteile aufweisen. Diese können z. B. durch einen Ionenaustausch mit den austauschbaren Kationen in den Zeolithen eingebaut werden. Beispielhaft sei hier aufgeführt der Austausch mit seltenen Erden, wie z. B. Gallium, Indium oder Lanthan, oder mit Nickel, Kobalt, Kupfer, Zink oder Silber.

Darüber hinaus können auch synthetische Zeolithe, in welche andere Atome, wie z. B. Bor oder Phosphor, die durch Kopräzipitation in das Gitter miteingebaut sind, erfindungsgemäß eingesetzt werden.

Der Konzentration der zu entfernenden Katalysatorgifte kommt zwar keine prinzipielle, wohl aber eine wirtschaftliche Bedeutung zu, denn deren Konzentration bestimmt das benötigte Volumen an erfindungsgemäß eingesetztem Molekularsieb pro Zeiteinheit. Im Normalfall wird es günstiger sein, mit Mitteln gemäß dem Stand der Technik Katalysatorgifte auf Gehalte bis zu etwa 1 000 Gew.-ppm zu reduzieren und das so an Katalysatorgiften abgereicherte Einsatz-Kohlenwasserstoff-Gemisch über das erfindungsgemäß eingesetzte Molekularsieb zu leiten. Aus diesem Grunde werden aus dem Einsatz-Kohlenwasserstoff-Gemisch vor dem Überleiten desselben über das Molekularsieb mit einem Porendurchmesser von größer als 4 Angström bis 15 Angström im wesentlichen Wasser, Alkohole, Stickstoff-, Schwefel- und Halogenverbindungen entfernt.

Hierbei kann die grobe Entfernung von Wasser und/oder Methanol aus dem Einsatz-Kohlenwasserstoff-Gemisch vorzugsweise mit Hilfe eines Molekularsiebes mit einem Porendurchmesser von bis zu 4 Angström erfolgen. Eine andere Verfahrensweise ist, daß Wasser und/oder Methanol und/oder Ethanol aus dem Einsatz-Kohlenwasserstoff-Gemisch durch Azeotropdestillation entfernt werden. Die letztere Verfahrensweise ist besonders geeignet für Einsatz-Kohlenwasserstoff-Gemische, die aus einer Methyl-tertiär-Butylether-Anlage oder einer tertiär-Amyl-Methylether-Anlage stammen, da bei der Azeotropdestillation gleichzeitig der Dimethylether, ein Nebenprodukt der Ethersynthese, mit abgetrennt wird.

Sind mehrfach ungesättigte Kohlenwasserstoffe im Einsatz-Kohlenwasserstoff-Gemisch vorhanden, so müssen diese aus demselben vor der Oligomerisierung entfernt werden.

Sie werden aus dem Einsatz-Kohlenwasserstoff-Gemisch durch eine selektive Hydrierung, z. B. gemäß EP-PS 81 041 und DE-PS 15 68 542, entfernt, bevorzugt durch eine selektive Hydrierung bis auf einen Restgehalt von unter 5 Gew.-ppm.

Die Entfernung der mehrfach ungesättigten Kohlenwasserstoffe aus dem Einsatz-Kohlenwasserstoff-Gemisch kann sowohl vor dem Überleiten desselben über das erfindungsgemäß eingesetzte Molekularsieb als auch nachher erfolgen.

Quantitative Aussagen über den Abtrennungsgrad der Katalysatorgifte aus dem Einsatz-Kohlenwasserstoff-Gemisch am erfindungsgemäß eingesetzten Molekularsieb sind sehr schwierig zu treffen, weil die Natur der Katalysatorgifte oft nicht bekannt ist.

Bevorzugt leitet man das Einsatz-Kohlenwasserstoff-Gemisch vor der Oligomerisierung über ein Molekularsieb mit einem Porendurchmesser von 7 bis 13 Angström.

Manchmal kann es aus wirtschaftlichen Gründen vorteilhaft sein, zwei oder mehrere erfindungsgemäß eingesetzte Molekularsiebe hintereinanderzuschalten. Man kann also auch das Einsatz-Kohlenwasserstoff-Gemisch vor der Oligomerisierung über zwei oder mehrere hintereinandergeschaltete Molekularsiebe mit einem Porendurchmesser von größer als 4 Angström bis 15 Angström leiten.

Das Überleiten des Einsatz-Kohlenwasserstoff-Gemisches über das erfindungsgemäß eingesetzte Molekularsieb kann sowohl in der Gasphase als auch in der Flüssigphase sowie in der Gasflüssigmischphase erfolgen. Es werden Raumgeschwindigkeiten (WHSV = weight hourly space velocity) von 0,05 bis 100 1/h bevorzugt und von 1 bis 40 1/h besonders bevorzugt.

Man leitet das Einsatz-Kohlenwasserstoff-Gemisch vor der Oligomerisierung über das erfindungsgemäß eingesetzte Molekularsieb bei Drücken von 1 bis 200 bar abs., bevorzugt bei Drücken von 1 bis 50 bar abs. und bei Temperaturen von 0 bis 200 °C, bevorzugt bei Temperaturen von 20 bis 160 °C.

Die Strömungsrichtung des Einsatz-Kohlenwasserstoff-Gemisches über das erfindungsgemäß eingesetzte Molekularsieb ist frei wählbar, bevorzugt von unten nach oben.

In der Regel bleibt die Schutzwirkung des erfindungsgemäß eingesetzten Molekularsiebs für den nachgeschalteten Oligomerisierungskatalysator für lange Zeit erhalten. Beobachtet man aber nach einer gewissen Zeit einen Durchbruch der Katalysatorgifte durch das erfindungsgemäß eingesetzte Molekularsieb, immer spätestens erkennbar an einem gewissen Rückgang des Olefinumsetzungsgrades, so kann man auf ein Reserve-Molekularsieb umschalten und kann zwischenzeitlich das erschöpfte regenerieren.

Die Häufigkeit der Regenerierung des erfindungsgemäß eingesetzten Molekularsiebes hängt u. a. von der Größe der Molekularsiebbetten, den Betriebstemperaturen sowie der Konzentration der Katalysatorgifte ab.

Man kann die erfindungsgemäß eingesetzten Molekularsiebe nach bekannter Weise regenerieren, z. B. mit einem inerten Regenerationsgas, wie z. B. Stickstoff oder Wasserstoff, oder einem gasförmigen Kohlenwasserstoff, wie z. B. Methan oder n-Butan, bei Temperaturen von etwa 160 bis 250 °C.

Die Erfindung wird durch die folgenden Beispiele näher erläutert:

### Beispiel 1

Es wird eine 15 gew.-%ige Lösung von Ethylen in n-Butan hergestellt. Diese Lösung wird bei Raumtemperatur über ein Adsorptionsbett, bestehend aus einem Molekularsieb mit einem Porendurchmesser von 3 Angström, mit einer LHSV von 5 l Lösung/l Adsorbens · h geleitet, und anschließend wird das noch in Spuren vorhandene Acetylen an einem Katalysator auf Basis von 0,5 Gew.-% Pd an Al₂O₃ mit einer WHSV von 20 h⁻¹ selektiv hydriert.

Dann wird die so vorbehandelte Lösung bei einer Temperatur von 25 °C und einem Druck von 50 bar abs. über ein Molekularsieb vom Typ 13 X der Firma Bayer AG (Porendurchmesser 9 Angström) mit einer WHSV von 4 · h⁻¹ geleitet. Irgendwelche Katalysatorgifte lassen sich weder vor noch nach dem Molekularsieb vom Typ 13 X der Firma Bayer AG nachweisen. Die so aufbereitete Lösung wird nun bei einer Temperatur von 70 °C, einem Druck von 50 bar und einer WHSV von 4 · h⁻¹ an einem nickelausgetauschten Montmorillonit (Montmorillonit der Firma Fluka AG, Darstellung: J. R. Sohn, H. B. Park, J. kor. chem. Soc. 26(5), S. 282 ff, 1982) oligomerisiert. Der Ethylenumsatz beträgt über eine Woche unverändert 99 %.

### Vegleichsbeispiel 1

Die Ethylenoligomerisierung erfolgt analog zu Beispiel 1, jedoch unter Verzicht auf das Molekularsieb vom Typ 13 X der Firma Bayer. Pro Tag sinkt der Ethylenumsatz um etwa 0,5 % und beträgt nach einer Woche nur mehr 95,5 %.

### Beispiel 2

Raffineriepropen mit einem Gehalt von etwa 75 Gew.-% Propen, in dem sich 0,45 Gew.-ppm Schwefel nachweisen lassen, wird analog zu Beispiel 1 aufbereitet und anschließend oligomerisiert, jedoch wird das Raffineriepropen vor der Oligomerisierung über einen mit Kupfer ausgetauschten X-Zeolithen mit einem Gehalt von 4,5 Gew.-% Cu (Porendurchmesser 8 Angström vor dem Austausch mit Kupfer), bei einer Temperatur von 120 °C, einem Druck von 50 bar abs. und mit einer WHSV von 0,75 · h⁻¹ geleitet. Nach Passieren dieses Cu-Zeolithen beträgt der Schwefelgehalt des Raffineriepropens nur mehr 7 Gew.-ppb. Zur Oligomerisierung dient ein Nickel-Aluminium-Siliziumoxid-Katalysator mit 15,3 Gew.-% NiO. 9,3 Gew.-% Al₂O₃ und 75,4 Gew.-% SiO₂, der gemäß DE-PS 20 51 402 durch Kopräzipitation von Ni-(NO₃)₂ mit Natriumsilikat in Gegenwart von kolloidalem Aluminiumoxid hergestellt wurde. Bei einer WHSV von 5 · h⁻¹, einem Druck von 50 bar abs. und einer Temperatur von 60 °C beträgt der Propenumsatz 53,5 %, der auch noch nach vier Wochen mit 53,0 % de facto unverändert ist.

### Vergleichsbeispiel 2

Raffineriepropen wird analog zu Beispiel 2 aufbereitet und oligomerisiert, allerdings ohne den mit Kupfer ausgetauschten X-Zeolithen. Nach nur einwöchiger Betriebsdauer ist der Propenumsatz auf 21,5 % abgefallen.

### Beispiel 3

Aus einem C₄-Kohlenwasserstoff-Schnitt aus einer MTB-Anlage mit 75 Gew.-% an n-Butenen wird Isobutan destillativ abgetrennt. Gleichzeitig damit wird jede Restfeuchte sowie der gesamte Gehalt an Methanol und Dimethylether über Kopf abgezogen. Das im Sumpf anfallende n-Bufenkonzentrat mit einem Gehalt von 79 Gew.-% n-Buten, 0,05 Gew.-% Isobuten und 0,7 Gew.-ppm tert.-Butylalkohol und 1,1 Gew.-ppm Methyl-tert.-Butylether wird analog zu Beispiel 1 selektiv hydriert, über ein Molekularsieb vom Typ 13 X der Firma Union Carbide (Porendurchmesser 10 Angström) bei einer Temperatur von 20 °C, einem Druck von 50 bar abs. und einer WHSV von 6 h⁻¹ geleitet und anschließend bei einer Temperatur von 140 °C, einem Druck von 20 bar abs. und einer WHSV von 6 · 1/h oligomerisiert. Nach dem Molekularsieb vom Typ 13 X der Firma Union Carbide beträgt der Gehalt an tert.-Butylalkohol 40 Gew.-ppb und der an Methyl-tert.-butylether 90 Gew.-ppb.

Der zur Oligomerisierung eingesetzte Katalysator wird wie folgt hergestellt:
500 g eines Molekularsiebes vom Typ 13 X der Firma Bayer AG werden mit 1 l einer 1molaren Nickelnitratlösung überschichtet und 6 Stunden lang bei einer Temperatur von 80 °C gehalten und gelegentlich geschüttelt. Danach wird die Lösung abdekantiert und der Katalysator für 5 Stunden bei einer Temperatur von 120 °C getrocknet und anschließend für 48 Stunden bei einer Temperatur von 350 °C unter Stickstoffatmosphäre kalziniert. Der fertige Oligomerisierungskatalysator enthält 9,7 Gew.-% Nickel.

Der Butenumsatz beträgt nach einer Einlaufphase von 3 Tagen 32 % und bleibt auch nach weiteren 14 Tagen unverändert.

### Vergleichsbeispiel 3

Die Oligomerisierung wird analog zu Beispiel 3 durchgeführt, aber anstelle des Molekularsiebes vom Typ 13 X der Firma Union Carbide wird ein Molekularsieb vom Typ 4 A der Firma Bayer AG (Porendurchmesser 4 Angström) verwendet. Der anfänglich gleiche Butenumsatz ist nach 14 Tagen auf 20 % abgefallen.

## Patentansprüche

1. Verfahren zur Oligomerisierung von Olefinen mit 2 bis 8 Kohlenstoffatomen oder deren Mischungen, die in einem Kohlenwasserstoff-Gemisch enthalten sind, in der Flüssigphase an einem heterogenen nickelhaltigen Katalysator bei Temperaturen von 0 bis 200°C und bei Drücken von 1 bis 70 bar abs.,
dadurch gekennzeichnet,
daß man das Einsatz-Kohlenwasserstoff-Gemisch vor der Oligomerisierung bei Drücken von 1 bis 200 bar abs. und bei Temperaturen von 0 bis 200°C über ein Molekularsieb mit einem Porendurchmesser von größer als 4.10⁻¹⁰ bis 15.10⁻¹⁰ m (4 Angström bis 15 Angström) leitet und die mehrfach ungesättigten Kohlenwasserstoffe aus dem Einsatz-Kohlenwasserstoff-Gemisch vor der Oligomerisierung durch eine selektive Hydrierung entfernt, wobei aus dem Einsatz-Kohlenwasserstoff-Gemisch vor dem Überleiten desselben über das Molekularsieb mit einem Porendurchmesser von größer als 4.10⁻¹⁰ bis 15.10⁻¹⁰ m (4 Angström bis 15 Angström) im wesentlichen Wasser, Alkohole, Stickstoff-, Schwefel- und Halogenverbindungen entfernt werden.

2. Verfahren nach Anspruch 1,
durch gekennzeichnet,
daß die Olefine im Einsatz-Kohlenwasserstoff-Gemisch an einem nickel-, silicium- und aluminiumhaltigen Katalysatorfestbett oligomerisiert werden.

3. Verfahren nach den Ansprüchen 1 und 2,
dadurch gekennzeichnet,
daß die mehrfach ungesättigten Kohlenwasserstoffe aus dem Einsatz-Kohlenwasserstoff-Gemisch durch eine selektive Hydrierung bis auf einen Restgehalt von unter 5 Gew.-ppm entfernt werden.

## Claims

1. Process for the oligomerization of olefins with 2 to 8 carbon atoms or their mixtures, which are contained in a hydrocarbon mixture, in the liquid phase on a heterogeneous nickel-containing catalyst at temperatures from 0 to 200°C and at pressures from 1 to 70 bar abs.,
characterized in that,
prior to oligomerization, the feed hydrocarbon mixture is passed over a molecular sieve having a pore diameter from greater than 4.10⁻¹⁰ up to 15.10⁻¹⁰ m (4 Angström units up to 15 Angström) units at pressures from 1 to 200 bar abs. and at temperatures from 0 to 200°C and the polyunsaturated hydrocarbons are removed from the feed hydrocarbon mixture prior to oligomerization through selective hydrogenation, wherein water, alcohols, nitrogen, sulphur and halogen compounds are essentially removed from the feed hydrocarbon mixture prior to the passing of the same over the molecular sieve having a pore diameter from greater than 4.10⁻¹⁰ up to 15.10⁻¹⁰ m (4 Angström units up to 15 Angström) units.

2. Process according to claim 1,
characterized in that
the olefins in the feed hydrocarbon mixture are oligomerized on a nickel-, silicon- and aluminium-containing catalyst fixed bed.

3. Process according to claims 1 and 2,
characterized in that
the polyunsaturated hydrocarbons are removed from the feed hydrocarbon mixture by selective hydrogenation until the content remaining is less than 5 wt. ppm.

## Revendications

1. Procédé pour l'oligomérisation d'oléfines avec de 2 à 8 atomes de carbone ou de leurs mélanges, qui sont contenus dans un mélange d'hydrocarbures dans la phase liquide sur un catalyseur hétérogène contenant du nickel à des températures de 0 à 200°C et à des pressions absolues de 1 à 70 bars ,
caractérisé on ce,
qu'on fait passer le mélange d'hydrocarbures et de charge avant l'oligomérisation sur un tamis moléculaire ayant un diamètre de pores plus grand que 4.10⁻¹⁰ à 15.10⁻¹⁰ m (4 à 15 angströms) à des pressions absolues de 1 à 200 bars et à des températures de 0 à 200°C et on élimine les hydrocarbures plusieurs fois non-saturés du mélange d' hydrocarbures et de charge avant l'oligomérisation, par uns hydrogénation sélective, en éliminant du mélange d'hydrocarbures et de charge avant le passage de celui-ci sur le tamis moléculaire avec un diamètre de pores plus grand que 4.10⁻¹⁰ à 15.10⁻¹⁰ m (4 à 15 angströms), essentiellement de l'eau, des alcools, des composés d'azote, de soufre et d'halogènes.

2. Procédé selon la revendication 1,
caractérisé on ce que
les oléfines sont oligomérisées dans le mélange d'hydrocarbures et de charge sur un lit fixe de catalyseur contenant du nickel, du silicium et de l'aluminium.

3. Procédé selon les revendications 1 et 2,
caractérisé en ce que
les hydrocarbures plusieurs fois nonsaturés sont éliminés du mélange de mise en oeuvre hydrocarbures par une hydrogénation sélective jusqu'à un contenu résiduel inférieur à 5 ppm en poids.
